# EUROPEAN PATENT SPECIFICATION

(11) **EP 1 855 731 B1**
(45) Date of publication and mention of the grant of the patent: **05.08.2009**
(21) Application number: 06726369.9
(22) Date of filing: 10.03.2006
(51) Int. Cl.: A61L 15/44, A61L 26/00

(54) **IMPROVEMENTS RELATING TO SKIN DRESSINGS**
VERBESSERUNGEN IM ZUSAMMENHANG MIT HAUTVERBÄNDEN
AMELIORATIONS APPORTEES AUX PANSEMENTS POUR LA PEAU

(30) Priority: 11.03.2005 GB 0505035
(43) Date of publication of application: 21.11.2007
(73) Proprietor: Insense Limited, Sharnbrook, Bedford MK44 1LQ (GB)
(72) Inventor: JEZEK, Jan, Northants NN9 6TE (GB); WATSON, Lynne Patricia, Bedfordshire MK43 9JT (GB)
(74) Representative: Willett, Christopher David
(86) International application number: PCT/GB2006/000873
(87) International publication number: WO 2006/095193

(56) References cited:
- WO-A-98/20015
- WO-A-03/017989

## Description

### Field of the. Invention

This invention relates to skin dressings for application to a part of a human or animal body for treatment of skin (for therapeutic or cosmetic purposes), and relates particularly (but not exclusively) to wound dressings for treatment of compromised skin, particularly skin lesions, i.e. any interruption in the surface of the skin, whether caused by injury or disease, including skin ulcers, burns, cuts, punctures, lacerations, blunt traumas, acne lesions, boils etc. The term "skin dressing" covers dressings such as patches, plasters, bandages and gauze etc. for use in connection with transdermal delivery of agents. The term also includes material in amorphous or liquid form. The term covers dressings for application to body surfaces generally, including internal and external tissues, particularly the skin including the scalp. The invention is based on the beneficial properties of nitric oxide (NO).

### Background to the Invention

### Physical and chemical properties of nitric oxide

Nitric oxide (NO) is a short-lived, unstable gaseous substance. Its instability is due to the unpaired electron of nitrogen:

•N̅=O

As an unstable substance with an unpaired electron, nitric oxide can be described as a free radical. However, compared with typical free radicals (e.g. hydroxyl radical or superoxide), whose life-time is in the order of milliseconds, nitric oxide is relatively stable. Typically, it is converted to a more stable chemical species within seconds of its production. Thus, for example, if gaseous nitric oxide contacts air, it reacts rapidly with oxygen to generate nitrogen dioxide (which is a brown gas) as follows:

2 NO + O₂ → 2 NO₂ →N₂O₄ (1)

Under some conditions, for instance in pure gaseous state, NO can be stored without significant losses for a very long time. It is also relatively stable in pure deoxygenated aqueous solutions.

NO is a very hydrophobic compound and its solubility in water is therefore limited. maximum solubility in water achievable under normal conditions is approximately 1.7 mM, with the solubility being similar to that of oxygen.

In aqueous solutions, the oxidation of dissolved nitric oxide by dissolved oxygen occurs, as shown in the reaction scheme below. Nevertheless, given the rate constants and low concentrations of dissolved NO and O₂ this reaction is not as rapid as in the gaseous state, where the concentration of oxygen is very high. However, the reaction is accelerated in heterogeneous environment containing water and lipids. In pure hydrophobic environments (e.g. lipid membranes) the reaction is accelerated as much as 300 fold. Once produced, nitrogen dioxide reacts rapidly with another molecule of nitric oxide giving rise to dinitrogen trioxide (N₂O;). N₂O₃ is a potent nitrosating agent capable of converting thiols into nitrosothiols. On hydration, N₂O₃ produces nitrous acid which dissociates to nitrite. Nitrite is oxidised in the presence of oxygen to nitrate.

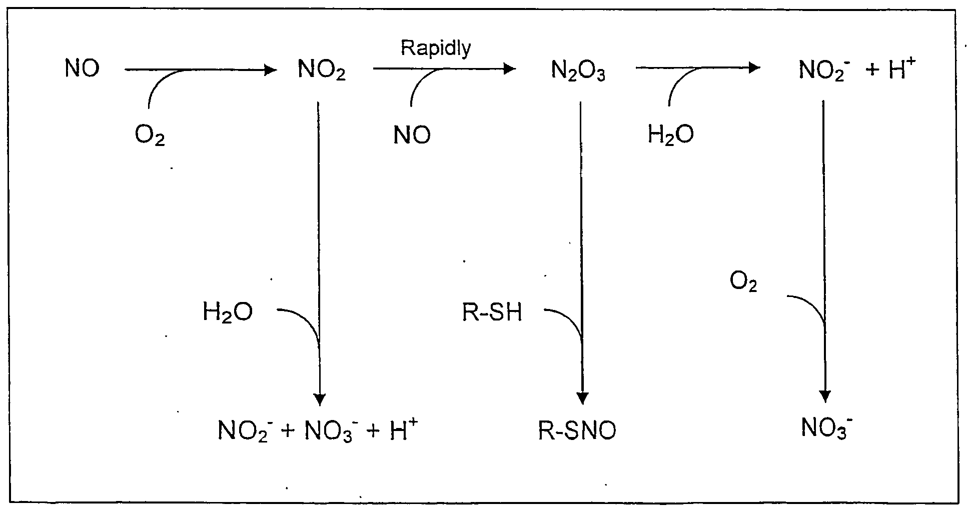

### Nitric oxide and free radicals

Although nitric oxide can be described as a free radical (see above) it is also an important scavenger of another potent free-radical called superoxide (O₂-). Reaction of nitric oxide with superoxide results in generation of peroxynitrite:

NO + O₂ →O = N - O- O⁻ (2)

Peroxynitrite is a powerful oxidant and a nitrating agent. During its short life it may function as a toxic chemical by oxidising, for example, parts of cell membranes and thus killing the cell. Peroxynitrite is thus a useful tool in the fight against infectious bacteria. Importantly, its potency to harm the cells of the host is minimal due to the rapid isomerisation reaction that converts peroxynitrate to nitrate:

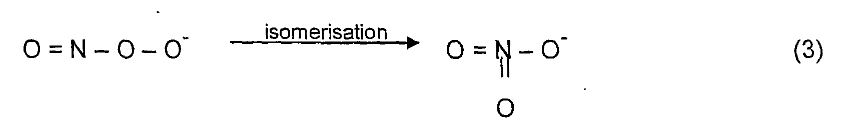

Any excess of peroxynitrate thus becomes a benign species (nitrate) that is ideal for excretion in urine. This prevents a build-up of peroxynitrate capable of causing serious harm to the cells of the host.

### S-Nitrosothiols

S Nitrosothiols (sometimes referred to simply as nitrosothiols) are compounds capable of releasing nitric oxide. S-nitrosothiols can be produced by nitrosating thiols using either N₂O₃ (equation 4) or nitrosonium cation (equation 5) as the nitrosating agent:

R-SH + N₂O₃ → R-SNO + NO₂⁻ + H⁺ (4)

R-SH + NO⁺ →R-SNO + H⁺ (5)

Whilst the process using N₂O₃ as the nitrosating species is very significant in vivo the second process is useful for production of nitrosothiols *in vitro*. Nitrosonium cation can be generated from nitrite at acidic pH:

NO₂ + 2 H⁺ ⇄ NO⁺ + H₂O (6)

S-nitrosothiols can thus be easily produced in laboratory by mixing a thiol (e.g. glutathione) with a source of nitrite (e.g. potassium nitrite) in acidic solution. The reaction proceeds at pH <6, the rate of the reaction increasing with the acidity of the solution:

R-SH + NO₂ +H⁺ → R-SNO + H₂O (7)

Nitrosothiols can release free nitric oxide by spontaneous decomposition:

2 O=N-S-R → 2 NO + R-S-S-R (8)

The rate of decomposition varies considerably depending on the side chain of the thiol. For example, whilst nitrosocysteine can be totally decomposed within minutes under normal conditions, it takes hours/days to achieve 100% decomposition of nitrosoglutathione. The decomposition is generally accelerated in the presence of CU²⁺ and Hg²⁺ .

WO 98/20015 discloses a compound comprising an S-nitrosothiol group linked via an intervening moiety to a mono-, di-, or trisaccharide moiety, the intervening moiety stabilising the S-nitrosothiol group, slowing down its degradation. Transdermal patches are disclosed, with the S-nitrosothiol being in active form, i.e. functioning to generate nitric oxide without requiring activation.

Nitrosothiols are also able to donate nitric oxide directly onto another thiol group. This process, which is called trans-nitrosation, is quite common *in vivo:*

R₁-SNO + R₂-SH → R₁-SH + R₂-SNO

### Nitric oxide in biological systems

### Nitric oxide synthase

Nitric Oxide Synthase (NOS) is the enzyme that generates NO *in vivo* from L-arginine:

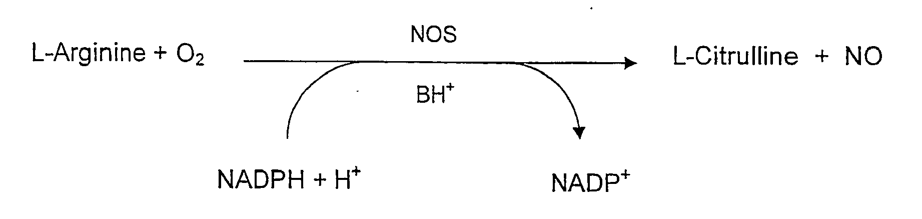

Apart from the substrates (L-arginine and oxygen), the enzyme requires the presence of cofactors nicotinamide adenine dinucleotide phosphate (NADPH) and tetrahydrobiopterine (BB)

The enzyme exists in three different isoforms. Each isoform synthesises NO but does so under different conditions.

NOS1 (or nNOS) is the neural isoform which can be found in neurons. Nitric oxide generated by this isoform is involved in synaptic transmission, the processing of nervous information across gaps between neurons.

NOS2 (or iNOS) is an inducible form which is produced by macrophages. NOS2 takes several hours to be mobilised and the response is due to an injury or infectious process. This enzyme generates extremely high concentrations of NO, in part to kill bacteria and in part to initiate tissue repair processes. In other words, when the body mounts an inflammatory response to injury, macrophages are attracted to the site of injury where they locally produce high concentrations of NO (100 to 1000 times normal). Unlike NOS1, which is active at all times as part of normal neurotransmission, there must be something abnormal (a wound, tissue damage, hypoxia, bacterial infection, etc.) to induce iNOS.

The third isoform is NOS3 (or eNOS). This isoform is active at all times and is found in endothelial cells (the cells that line the inner surface of blood vessels and lymph ducts). NO produced by eNOS maintains the diameter of blood vessel so that perfusion of tissues (skin, muscle, nerves, and bone) is maintained at optimal levels. In addition, eNOS mediated NO causes neovascularisation, which is the growth of new blood vessels. This is especially important in healing an ulcer or wound on the skin.

### Biolorgical effects of nitric oxide

Nitric oxide has a multitude of effects in living tissues. The mechanism of these effects is nearly always based on interaction of nitric oxide either with metal component (typically iron) or with thiol groups of key enzymes and other proteins. Depending on the particular enzyme, such interaction can lead to either activation or inhibition of the enzyme. An example of an effect based on the activation of the enzyme is that of vasodilatation: nitric oxide binds to the haem iron of the enzyme guanylate cyclase, which results in conformational change exposing the catalytic site of the enzyme. This leads to catalytic conversion of GTP to cGMP. This conversion initiates the whole cascade of reactions leading to protein phosphorylation and muscle relaxation (vasodilatation).

Other effects based on activation of enzymes or growth factors by nitric oxide include stimulation of cell division (proliferation) and cell maturation, stimulation of cell differentiation and formation of cell receptors, neovascularisation, formation of fibroblasts in the wound and thereby enhancement of collagen formation, etc. In short, nitric oxide is the pivotal point of regulating cellular growth and differentiation.

Nevertheless, nitric oxide is also capable of causing the opposite effect, namely cellular death. This can be typically achieved by NO binding to the iron of the iron-sulphur clusters of vital enzymes (e.g. enzymes involved in respiratory chain such as cytochrome c) leading to the enzyme inhibition and subsequent cellular death. There is also some experimental evidence suggesting that NO can stimulate the gene responsible for the process called apoptosis or programmed cell death. Apoptosis is the continuous process involved in the daily maintenance of mature organs by removing the ageing or faulty cells that are being replaced by fresh ones.

The involvement of nitric oxide in a great number of processes of fundamental importance, as outlined briefly above, makes this molecule the pivotal point in regulation of the growth and maintenance of healthy living tissues. Its importance in repair of damaged tissues is even greater and is briefly outlined in the following section.

US 6,103,275 discloses a biocompatible system for generating nitric oxide by bringing together a nitrite, a reductant and a particular acid. The nitrite and acid are typically kept separate until the moment of use.

WO 03/017989 discloses a skin dressing comprising a nitric oxide donor together with a nitrosothiol precursor. During use of the dressing the donor and precursor enter the wound site and generate nitric oxide in the wound which reacts with the precursor in the wound to generate nitrosothiols *in vivo.*

### Nitric oxide in wound healing

Both iNOS and eNOS play an important role in repair mechanisms of wound healing. In the first stage of normal wound healing process, NO is generated from iNOS in order to (i) fight infection, (ii) remove irreversibly damaged necrotic tissue, and (iii) initiate the neovascularisation. This is often referred to as the inflammatory stage of wound repair. Typically, this phase lasts for approximately 10 days. By the end of this phase the granulation tissue is robust. The neovascularisation results in increase of activity of eNOS that gradually takes over from iNOS.

The increased activity of eNOS causes further neovascularisation and vasodilation to continue the healing process. Vasodilation increases blood supply both to the repairing tissues and away from the damaged tissue. The latter removes metabolic waste products, reduces oedema, and prevents swelling that would otherwise compress capillaries. In the absence of adequate blood supply, tissue will remain hypoxic and heal only slowly, if at all. Moreover, since iNOS is produced in large part by white blood cells, vasodilation permits delivery of additional blood cells to the area that needs to be protected from infection.

In diabetic patients, however, eNOS activity is often well below normal so these patients cannot produce NO at normal levels and the wound healing is thus retarded. The availability of NO is further lowered in diabetic patients by increased production of superoxide that scavenges the NO, and by production of dimethylarginine (due to kidney disfunction), which is the competitive inhibitor of NOS.

Insufficient vascularisation and excessive oxidative stress (i.e. high production of superoxide) also limits the beneficial effects of NO in the healing of venous ulcers.

### Summary of the Invention

The present invention provides a skin dressing adapted, on activation, to release one or more S-nitrosothiols, wherein the one or more S-nitrosothiols are generated by reacting together reagents, comprising a nitrite and a thiol, in the dressing.

The dressing is therefore inactive, in the sense that it does not release one or more w S-nitrosothiols until it is activated. The invention thus relates to an inactive skin dressing, that is, a dressing in a form in which it does not function to release one or more S-nitrosothiols. However, the dressing can be activated, as discussed below, to a form in which it functions to release one or more S-nitrosothiols. Prior to use, the dressing is kept in inactive condition, being activated when required for use.

S-nitrosothiols decompose spontaneously to produce nitric oxide and the oxidised form of the thiol, as discussed above and as set out in equation 8 above. The dressing, on activation, typically in use on skin, thus functions as a nitric oxide donor, generating nitric oxide from the released S-nitrosothiol, typically on or in the vicinity of the skin being treated, e.g. with nitric oxide being released into a wound. Nitric oxide has beneficial effects on tissues, particularly in wound healing, as discussed above. Nitric oxide also functions as a vasodilator, causing blood capillaries in the vicinity to open up. This effect can enhance transdermal delivery of materials, e.g. hormones, analgesics etc, by accelerating delivery and uptake of the materials. The dressing can thus be used as an adjuvant for transdermal delivery, typically by having a composite dressing or patch, plaster, bandage, gauze etc. also including material for delivery.

Nitric oxide may be of particular use in alleviating a condition known as Raynaud's Syndrome.

Also of interest to the inventors is a means of treating or preventing restenosis (narrowing) and/or thrombosis of blood vessels following surgical procedures: physical damage to, or removal of, the endothelium during percutaneous transluminal angioplasty (PCTA) is a major contributory factor in the high incidence of restenosis following PCTA (Langford et al, Lancet 344, 1458-1460).

Suitable S-nitrosothiols include S-nitrosoglutathione (preferably S-nitroso-L-glutathione, as this is the physiologically important version), S-nitrosocysteine, S-nitroso-N-acetylcysteine, S-nitrosocaptopril, S-nitrosomercaptoethylamine, S-nitroso-3-mercaptopropanoic acid, S-nitroso-D-thioglucose and S-nitroso-N-acetyl-D, L-penicillamine. S-nitrosoglutathione is currently preferred, because of its relatively slow rate of decomposition to generate nitric oxide, resulting in satisfactory stability of the S-nitrosothiol in the dressing and consequential slow release of nitric oxide at an appropriate rate for skin benefits.

The dressing includes one or more dressing components including one or more reagents (either an S-nitrosothiol or precursors thereof) that function to release the S-nitrosothiol (possibly after generation in the dressing) from the dressing on activation of the dressing. Prior to use, the dressing is kept in inactive condition to prevent premature release of S-nitrosothiol.

The dressing includes a nitrite, e.g. potassium nitrite, and a thiol, e.g. L-glutathione as reagents that react together in the dressing on activation to generate and release S-nitrosothiol. When brought together in acidic solution, the reagents react together to generate S-nitrosothiol, as set out in equation 7 above. The reagents are suitably provided in separate components of the dressing that are kept apart (e.g. in separate packages) until required for use. To activate the dressing in use, the two dressing components are brought into contact (in the presence of a source of water and protons, if required), resulting in production in the dressing of the S-nitrosothiol that is then released from the dressing.

Alternatively, reagents may react in the dressing to generate S-nitrosothiol before activation to release S-nitrosothiol as is discussed further below. In order to remain in inactive condition, the S-nitrosothiol should be in dry condition. On wetting of the dressing, the S-nitrosothiol is released. The dressing is thus readily activated by exposure to water; e.g. on contact with a moist wound bed. S-nitrosothiol is typically generated in the dressing by reaction of nitrite and thiol, as discussed above.

Suitable amounts of the reagents can be readily determined to produce desired amounts of S-nitrosothiols. In general, amounts of each reagent in the range 1-50 mM are likely to be appropriate.

The or each dressing component may be in the form of a layer, e.g. in the form of a sheet, slab or film, that may produce from an amorphous material, not having any fixed form or shape, that can be deformed and shaped in three dimensions, including being squeezed through a nozzle.

The or each dressing component conveniently comprises a carrier or support, typically in the form of a polymeric matrix. The carrier may be solid or amorphous, as discussed below.

The carrier or support conveniently comprises a hydrated hydrogel. A hydrated hydrogel means one or more water-based or aqueous gels, in hydrated form. A hydrated hydrogel thus includes a source of water, for activation of the dressing. A hydrated hydrogel can also act to absorb water and other materials exuded from a wound site, enabling the dressing to perform a valuable and useful function by removing such materials from a wound site. The hydrated hydrogel also provides a source of moisture, that can act in use to maintain a wound site moist, aiding healing.

Suitable hydrated hydrogels are disclosed in WO 03/090800. The hydrated hydrogel conveniently comprises hydrophilic polymer material. Suitable hydrophilic polymer materials include polyacrylates and methacrylates, e.g. as supplied by First Water Ltd in the form of proprietary hydrogels, including poly 2-acrylamido-2-methylpropane sulphonic acid (poly-AMPS) and/or salts thereof (e.g. as described in WO 01/96422), polysaccharides e.g. polysaccharide gums particularly xanthan gum (e.g. available under the Trade Mark Keltrol), various sugars, polycarboxylic acids (e.g. available under the Trade Mark Gantrez AN-169 BF from ISP Europe), poly(methyl vinyl ether co-maleic anhydride) (e.g. available under the Trade Mark Gantrez AN 139, having a molecular weight in the range 20,000 to 40,000), polyvinyl pyrrolidone (e.g. in the form of commercially available grades known as PVP K-30 and PVP K-90), polyethylene oxide (e.g. available under the Trade Mark Polyox WSR-301), polyvinyl alcohol (e.g. available under the Trade Mark Elvanol), cross-linked polyacrylic polymer (e.g. available under the Trade Mark Carbopol EZ-1), celluloses and modified celluloses including hydroxypropyl cellulose (e.g. available under the Trade Mark Klucel EEF), sodium carboxymethyl cellulose (e.g. available under the Trade Mark Cellulose Gum 7LF) and hydroxyethyl cellulose (e.g. available under the Trade Mark Natrosol 250 LR).

Mixtures of hydrophilic polymer materials may be used in a gel.

In a hydrated hydrogel of hydrophilic polymer material, the hydrophilic polymer material is desirably present at a concentration of at least 1%, preferably at least 2%, more preferably at least 5%, yet more preferably at least 10%, or at least 20%, desirably at least 25% and even more desirably at least 30% by weight based on the total weight of the gel. Even higher amounts, up to about 40% by weight based on the total weight of the gel, may be used.

Good results have been obtained with use of a hydrated hydrogel of poly-AMPS and/or salts thereof in an amount of about 30% by weight of the total weight of the gel.

By using a gel comprising a relatively high concentration (at least 2% by weight) of hydrophilic polymer material, the gel can function particularly effectively to take up water in use of the dressing, e.g. from serum exudates while in contact with a wound. Because the gel is an aqueous system, use of the dressing does not have the effect of inducing an overall dryness of the wound which would be undesirable. This is because water vapour pressure is maintained in the enclosed environment surrounding the skin in use of the dressing. The gel thus functions as an absorbent entity for the removal of moisture, e.g. wound exudate, that also provides a helpful background level of excess moisture.

The water-uptake capacity of a hydrated hydrogel, including a high concentration gel, enables the dressing to aid wound healing by removing substantial amounts of exudates, swelling-up as it does so. By using a carefully formulated, ready-hydrated gel, the wound is prevented from reaching a state of unhelpful dryness. Ready hydration also ensures the quick formation of an aqueous liquid interface between the dressing and the wound, thus preventing adhesion, which otherwise would interfere with easy lifting of the dressing when it has to be replaced. A good aqueous liquid interface between the wound and the dressing is also important in allowing any beneficial products carried in the gel to enter the wound through all of the available surface.

The hydrated hydrogel material is typically in the form of a solid layer, sheet or film of material that is typically cross-linked, and that may incorporate a mechanical reinforcing structure. The size and shape of the layer, sheet or film can be selected to suit the intended use of the dressing. Thicknesses in the range 0.05 to 5 mm, preferably 0.5 to 3 mm are particularly suitable.

Alternatively, the hydrated hydrogel may be in the form of an amorphous gel not having a fixed form or shape, that can be deformed and shaped in three dimensions, including being squeezed through a nozzle. Amorphous gels are typically not cross-linked or have low levels of cross-linking. A shear-thinning amorphous gel may be used. Such a gel is liquid when subjected to shear stress (e.g. when being poured or squeezed through a nozzle) but set when static. Thus the gel may be in the form of a pourable or squeezable component that may be dispensed, e.g. from a compressible tube or a syringe-like dispenser, comprising a piston and cylinder, typically with a nozzle of about 3 mm diameter. Such a gel may be applied in the form of a surface layer, or into a wound cavity as a fully conformable gel that fills the available space and contacts the wound surface.

A typical example of an amorphous gel formulation is: 15% w/w AMPS (sodium salt), 0.19% polyethylene glycol diacrylate and 0.01% hydroxycyclohexyl phenyl ketone, with the volume made up to 100% with analytical grade DI water. The reagents are thoroughly mixed and dissolved, then polymerised for between 30-60 seconds, using a UV-A lamp delivering approximately 100 mW/cm², to form the required hydrogel. This may be contained in plastic syringes from which the amorphous gel may then be dispensed from a syringe to a target site, as a surface layer or to fill a cavity.

While it is generally preferred to use a hydrated hydrogel as the carrier or support, the carrier or support may instead comprise material in dry condition, with the reagent typically present in a dried polymeric matrix.

Dry condition means that there is no free water in the material, such that no significant or measurable water loss occurs through evaporation under normal ambient conditions of temperature, pressure and humidity. Dry condition includes desiccated condition, which is an extra thoroughly dried condition. Desiccated condition means a condition maintained by storage in an environment enclosed by a moisture impermeable barrier, wherein the material is kept scrupulously free of water by means of an added desiccant.

Because the material is in dry condition the reagent is in stable condition and is retained in the material. The material can be stored under suitable conditions for an extended period of time, with the reagent remaining stable therein.

When the material is wetted, e.g. by contact with a source of water, the reagent is solubilised and released. Sufficient water is required to form a contact liquid junction between the material and a water source.

The reagent is typically incorporated in the solid material, being dispersed throughout the material. The solid material typically comprises a matrix with the reagent dispersed therein, preferably in a reasonably homogeneous manner.

The solid material preferably comprises a polymer material.

One preferred polymer material comprises polyvinyl alcohol (PVA). PVA has convenient and acceptable properties for skin treatment use, e.g. being non-toxic. PVA is also easy to handle and use, readily forming a film on drying of a PVA solution in water, with the resulting film being easy to handle. PVA is also readily available and cheap. Cross-linking is not required to form a solid material, e.g. in the form of a film, although cross-linking may optionally be employed. PVA is available in a wide range of grades based on molecular weight and degree of hydrolysis, which affect the physical properties of the material.

Appropriate grades of PVA can be readily selected to produce a polymer product having desired properties for a particular intended use. For example, for use in skin dressings, good results have been obtained by use of PVA with a molecular weight in the range 100,000 to 200,000, substantially fully hydrolysed (98-99% hydrolysed), e.g. in the form of code 36,316-2 from Aldrich, in non-cross-linked form.

Another suitable polymer material comprises polyvinylpyrrolidone (PVP). The properties of PVP are very similar to those of PVA, and PVP is also acceptable for skin treatment use. PVP is readily available in a range of different molecular weights. Appropriate grades of PVP can be readily selected. For example, good results have been obtained using a PVP having a molecular weight average of 360,000, e.g. in the form of code PVP360 from Sigma, in a non-crosslinked form.

Mixtures of polymer materials may be used.

The solid material is conveniently in the form of a sheet, layer or film, typically having a thickness in the range 0.01 to 1.0mm, preferably in the range 0.05 to 0.5mm.

The solid material may optionally include a support to provide rigidity when wet.

The solid material of the invention is conveniently made by mixing a solution of a polymer (e.g. an aqueous solution of PVA and/or PVP) and reagent, and drying the mixture to produce a solid material, e.g. forming film by a casting procedure. Suitable techniques are well known to those skilled in the art.

The polymer material or materials are suitably used in appropriate amounts that result in formation of a film, with the upper limit of concentration typically being dictated by the limit of solubility (generally in water) and the lower limit of concentration being the point at which a film does not form. For PVA code 36,316-2 from Aldrich, the limit of solubility in water is about 6% w/w, resulting in a concentration of PVA in the film prior to drying of about 5%.

While the use of a hydrated hydrogel, particularly poly-AMPS, as the carrier or support is generally preferred, practical difficulties arise in incorporating the thiol L-glutathione in a poly-AMPS hydrogel, so this reagent is instead generally provided in a carrier comprising dry material as discussed above, e.g. a dried PVA polymeric matrix.

Thus, in one preferred embodiment the invention comprises a first component comprising a layer of hydrated hydrogel, preferably poly-AMPS and/or salts thereof, containing a source of nitrite, e.g. potassium nitrite, and a second component comprising a dry polymeric matrix, preferably dried PVA, containing a thiol, e.g. L-glutathione. The first component may be used in contact with the skin, as the hydrated hydrogel has beneficial properties for skin contact, as discussed above, with the second component being placed on top of the first component. Provided the components are kept separate prior to use, the dressing remains in non-activated condition. However, when the two components are brought into contact, this has the effect of activating the dressing. The water in the hydrated hydrogel of the first component functions to provide a suitable aqueous environment, with L-glutathione, which is acidic, acting as a source of protons, providing the necessary acidic environment for reaction.

On activation of the dressing, nitrite starts diffusing from the first component (or primary layer) into the second component (or secondary layer), and the thiol diffuses in the opposite direction. Mixing of the nitrite with the thiol in acidic solution results in slow generation of S-nitrosothiol. If the thiol is L-glutathione, then the product of reaction is S-nitroso-L-glutathione. Once produced, the S-nitrosothiol is released from the dressing into the surrounding environment, e.g. into a wound bed, where it decomposes to produce nitric oxide, with consequential beneficial effects. These reactions are as illustrated below.

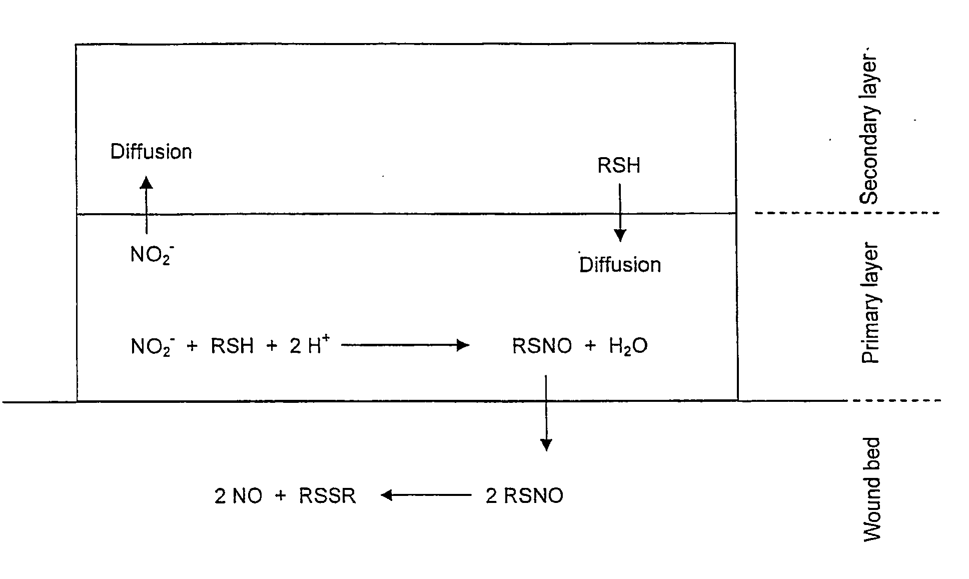

The preferred nitric oxide donor to be generated by the activated dressing is S-nitrosoglutathione (GSNO). The rate of GSNO production in aqueous environment containing glutathione and nitrite is pH dependent. Whilst the rate is negligible at pH 7 it can be observed at acidic pH (<6). pH 5 (or lower) is sufficient to drive the reaction forward at satisfactory rate. The pH of poly-AMPS hydrogel (with sodium counterion), the preferred material of the first component or primary layer, is approximately 7 and a source of protons is therefore needed in order to achieve pH 5 or less to drive the GSNO production forward. Glutathione (incorporated in the second component or secondary layer) is itself an acidic compound capable of donating protons to allow generation of GSNO in the activated dressing, so an additional source of protons is not essential.

The production of GSNO in the activated dressing peaks approximately 2 h after the activation (see Example 2 below) after which point the total amount of GSNO declines gradually due to utilisation of reagents (nitrite and glutathione) and slow decomposition of the GSNO. The rate of release of GSNO from the activated dressing was modelled using a piece of blank poly-AMPS hydrogel as the substitute of the wound bed (see Example 3 below).

The dressing may optionally include and/or generate on activation a (possibly additional) source of protons.

For example, an acid, e.g. lactic acid, more preferably an acidic buffer, e.g. citrate buffer, citrate-phosphate buffer etc., may be included in one or both of the first and second dressing components.

Incorporation of the additional source of protons allows a degree of control over the rate of S-nitrosothiol production inside the activated dressing. The rate of the production increases with the acidity of the dressing regulated by the buffer incorporated. Thus, for example, the rate of the S-nitrosothiol production will be slower if phosphate buffer (pH 5.5) is incorporated as the source of protons compared with incorporation of citrate buffer (pH 3).

Optionally, the source of protons can be used to activate the dressing by reducing the pH to a point where reaction of e.g. nitrite and thiol, can occur. For example nitrite and thiol could be kept together at a pH above, say, 7.0. Upon activation, the pH drops to initiate reaction to generate one or more S-nitrosothiols.

As a further possibility, protons may be generated in the dressing on activation, e.g. from an oxidase enzyme/substrate system. An oxidase enzyme catalyses reation of an appropriate substrate with oxygen to produce hydrogen peroxide and an acid, which dissociates to produce protons. Various enzyme/substrate pairs are disclosed in WO 03/090800. The preferred oxidase/substrate system is glucose oxidase and glucose. Glucose oxidase catalyses oxidation of glucose by oxygen to produce hydrogen peroxide and gluconic acid. Gluconic acid dissociates to produce gluconate anion and a proton and can thus serve as the source of protons:

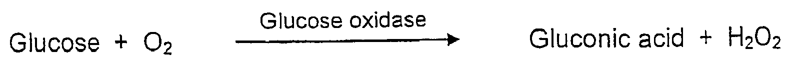

Gluconic acid ⇄ Gluconate + H⁺

The enzyme and corresponding substrate are conveniently incorporated in separate dressing components (which may correspond to or be different from the first and second components discussed above) so they are not in contact prior to activation of the dressing. However, on activation of the dressing, the enzyme and substrate are brought into communication permitting contact, resulting in generation of protons.

The substrate is conveniently incorporated in the first component or primary layer, and the enzyme is preferably incorporated in a component not in contact with the skin in use, e.g. the second component or secondary layer referred to above. Thus, in one preferred arrangement, the first dressing component or primary layer comprises a polymeric matrix (preferably poly-AMPS hydrated hydrogel) which contains a source of nitrite (preferably potassium nitrite) and the substrate for the oxidase enzyme (preferably glucose), and second dressing component or the secondary layer comprises a polymeric matrix (preferably dried PVA) which contains a thiol (preferably L-glutathione) and the oxidase enzyme (preferably glucose oxidase).

On activation, nitrite and glucose start diffusing from the primary layer into the secondary layer and the thiol diffuses in the opposite direction. The mobility of glucose oxidase in the polymeric matrix is very limited, so the enzyme will typicaly remain confined in the secondary layer. Whilst mixing of glucose with the glucose oxidase in the secondary layer results in generation of protons, mixing of the nitrite with the thiol results in generation of S-nitrosothiol. Protons generated in the secondary layer increase the rate of the S-nitrosothiol production inside of the dressing. Once produced, the S-nitrosothiol is released from the dressing into the surrounding environment where it decomposes to produce nitric oxide.

As a further possibility, the dressing can comprise three components: a first component or primary layer comprising a polymeric matrix (preferably poly-AMPS hydrogel) which contains a source of nitrite (preferably potassium nitrite) and the substrate for the oxidase enzyme (preferably glucose); a second component or secondary layer comprising a polymeric matrix (preferably dried PVA) which contains a thiol (preferably L-glutathione); and a third component or a tertiary layer comprising a polymeric matrix (preferably poly-AMPS hydrogel) which contains the oxidase enzyme (preferably glucose oxidase). The dressing can be activated by bringing all three layers together. The particular order in which the layers are assembled is not crucial. Nevertheless the preferred arrangement is that in which the secondary layer is sandwiched between the primary and the tertiary layer.

On activation, nitrite and glucose start diffusing from the primary layer into the secondary and tertiary layer and the thiol diffuses away from the secondary layer. The mobility of glucose oxidase in the polymeric matrix is very limited, so the enzyme will mostly remain confined in the tertiary layer. Whilst mixing of glucose with the glucose oxidase in the tertiary layer results in generation of protons mixing of the nitrite with the thiol results in generation of S-nitrosothiol. Protons generated in the tertiary layer spread across the entire dressing and increase the rate of the S-nitrosothiol production inside of the dressing. Once produced, the S-nitrosothiol is released from the dressing into the surrounding environment where it decomposes to produce nitric oxide. The reactions are as represented below.

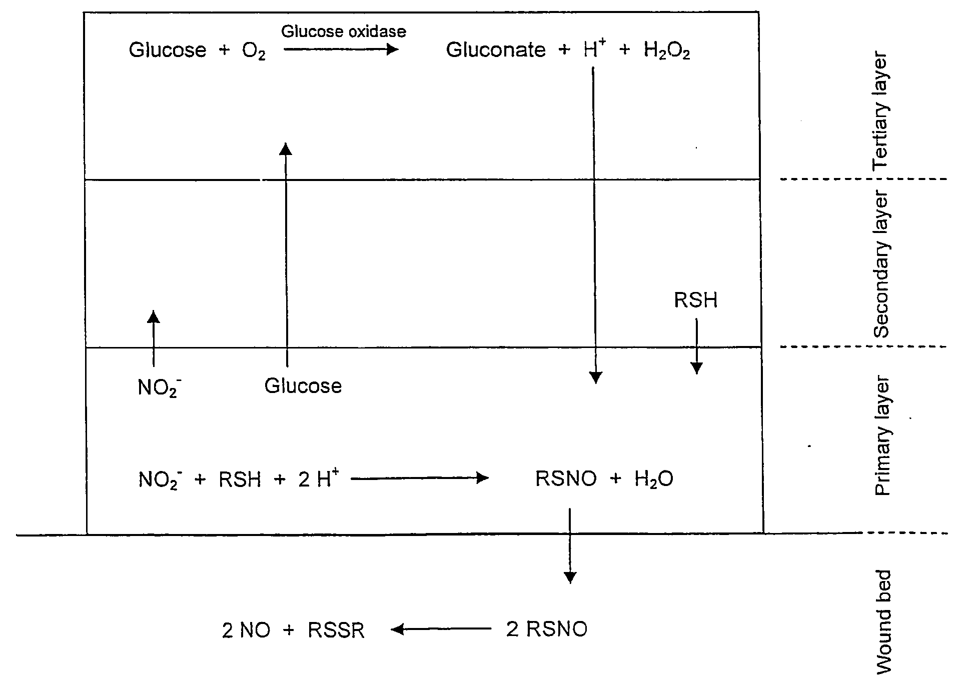

Instead of the dressing including reagents that react together in the dressing on activation to generate and release S-nitrosothiol, the dressing may include S-nitrosothiol (possibly pre-generated *in situ)* in inactive condition, with the S-nitrosothiol being released on activation of the dressing. The S-nitrosothiol (preferably S-nitroso-L-glutathione) is conveniently provided in a dressing component as discussed above, preferably in the form of a layer, e.g. in the form of a sheet, slab or film. The dressing component conveniently comprises a carrier or support, typically in the form of a polymeric matrix.

In order to remain in inactive condition, the S-nitrosothiol should be in dry condition. The carrier or support should also be in dry condition, with the S-nitrosothiol conveniently being present in a dried polymeric matrix. This is as described above, and conveniently comprises dried PVA. On wetting of the dressing, the S-nitrosothiol is released. The dressing is thus readily activated by exposure to water, e.g. on contact with a moist wound bed.

S-nitrosothiol is conveniently pre-generated in the dressing component, typically by reaction of nitrite and thiol as discussed above.

In one preferred embodiment of this type, the dressing comprises a layer of dried PVA containing pre-generated S-nitrosothiol. The layer is formed from PVA, a source of nitrite (preferably potassium nitrite) and a thiol (preferably L-glutathione). In a typical example of this embodiment both of these additives are added to the PVA solution prior to the drying. S-nitrosothiol (GSNO in the preferred case) is generated within the layer during the drying step. Nitrosothiols are known to be rather unstable in aqueous solutions. Nevertheless, GSNO was found very stable in the dried layer of PVA, especially if stored in a moisture-free atmosphere.

GSNO can be released from the layer simply by applying the layer onto a moist surface (e.g. wound bed). Once released, GSNO undergoes a slow decomposition to generate nitric oxide. The release of GSNO from the layer is relatively rapid. This can be demonstrated by applying the layer onto a moist skin, which results in rapid (within approximately 1 min) reddening of the skin due to dermal vasodilation. The reddening is totally reversible and disappears within several minutes after removal of the patch.

An additional layer consisting of a hydrated polymeric matrix (preferably poly-AMPS hydrogel) can be used in this embodiment (and generally in other embodiments) as a transition layer between the skin and the dried PVA layer to slow down the release of the nitric oxide donor into the surrounding environment. The hydrated polymeric matrix also functions as a source of water to activate the dressing.

The dressing may optionally include a source of water. This may be similar to the second component of the dressing described in WO 2004/108176, and conveniently comprises a hydrated poly-AMPS hydrogel.

In a preferred embodiment, the dressing comprises two components which are amorphous. The components can be in the form of e.g. a gel, semi-solid, paste, cream, lotion or liquid e.g. an aqueous solution. Hydrated hydrogels may be conveniently employed, as discussed above.

In embodiments of this type, each component preferably contains a reagent which, when brought together, activate to release one or more S-nitrosothiols. Preferably one component contains a nitrite and the other contains a thiol. Alternatively the nitrite and thiol could be kept together at a high enough pH to prevent reaction thereof, e.g. at a pH above 7, the second component containing a source of acidity. Another possibility is that one component contains anhydrous S-nitrosothiol and the second component contains water.

The two amorphous components are kept separate until it is desired to apply the dressing to a body surface. Conveniently they are packaged in a container having a nozzle, through which the amorphous components can be delivered. Preferably, the two components are packaged in a two compartment dispenser, preferably being operable to deliver both components simultaneously.

Preferred embodiments comprise two dressing components, one containing nitrite and the other containing thiol, e.g. glutathione. The two components can take a wide variety of material forms, as discussed above. However, the following examples of combinations are currently preferred:

| **Nitrite component** | **Thiol component** |
|---|---|
| Water | Dry PVA film |
| Water | PVA film with glycerol humectant |
| Viscous aqueous solution | Dry PVA film |
| Viscous aqueous solution | Suspension in glycerol |
| Water | Suspension in propylene glycol |
| Amorphous gel | Water |
| Water | Water |
| Amorphous gel | Amorphous gel |
| Water | Sheet hydrogel |
| Sheet hydrogel | Water |
| High water content sheet hydrogel | High water content sheet hydrogel |

then dressing optionally includes, or is used with, a skin contact layer, preferably comprising a hydrated hydrogel of poly-AMPS and/or salts thereof, as mentioned above.

The dressing optionally includes, or is used with, a covering or outer layer for adhering the dressing to the skin of a human or animal in known manner.

Dressings in accordance with the invention can be manufactured in a range of different sizes and shapes for treatment of areas of skin e.g. wounds of different sizes and shapes. Appropriate amounts of reagents for a particular dressing can be readily determined by experiment.

Dressing components are suitably stored prior to use in sterile, sealed, water-impervious packages, e.g. laminated aluminium foil packages. In the case of components comprising dry material, desiccant material is desirably included in the packages.

In use, the dressing component or components are removed from their packaging and located in appropriate order on the skin of a human or animal, e.g. over a wound or other region of skin to be treated for cosmetic or therapeutic purposes. The dressing may also be used as an adjuvant for transdermal delivery, as noted above. The dressing is activated, in the case of multiple component dressings, by bringing the components into contact, and in the case of dry dressings by bringing into contact with a source of water (e.g. from a wound), resulting in release from the dressing of one or more S-nitrosothiols (possibly after generation in the dressing after activation). S-nitrosothiols decompose spontaneously to produce nitric oxide, which has beneficial effects on tissues and also causes vasodilation.

In another aspect, the present invention provides a method of generating nitric oxide for therapeutic and/or cosmetic purposes on or in the vicinity of a body surface, the method comprising reacting a nitrite and a thiol in a dressing to generate one or more S-nitrosothiols which decompose spontaneously to deliver the nitric oxide.

The invention will be further described, by way of illustration, in the following Examples, and with reference to the accompanying drawings, in which:
Figure 1 is a graph of concentration of S-nitrosoglutathione (in mM) versus time (in minutes) showing the effect of pH on the rate of production and subsequent decomposition of S-nitrosoglutathione in solutions containing potassium nitrite (5mM) and L-glutathione (5
Figure 2 is a graph of GSNO concentration (in mM) versus time (in hours) showing the concentration profile of S-nitrosoglutathione in a dressing following activation of the dressing at time 0;
Figure 3 is a graph of GSNO concentration (in mM) versus time (in hours) showing the concentration profile of S-nitrosoglutathione in a dressing and in a blank hydrogel placed beneath the dressing following activation of the dressing at time 0;
Figure 4 is a graph of GSNO concentration (in mM) versus time (in hours) showing the oncentration profile of S-nitrosoglutathione in various dressing layers following activation of the dressing at time 0;
Figure 5 is a graph of GSNO concentration (in mg) versus time (in hours) showing the release of S-nitrosoglutathione from a dry PVA layer into a transition hydrogel layer and subsequently into another layer of hydrogel following activation of the dressing at time 0; and
Figure 6 is a schematic illustration of an embodiment of wound dressing in accordance with the invention.

### Examples

### Materials and Methods

### Chemicals & other materials

Water (conductivity < 10 µS cm⁻¹; either analytical reagent grade, Fisher or Sanyo Fistreem MultiPure)
AMPS (2-acrylamido-2-methylpropane sulphonic) sodium salt, 50% aqueous solution, hydrogel monomer - Lubrizol, AMPS 2405
AMPS (2-acrylamido-2-methylpropane sulphonic) ammonium salt, 50% aqueous solution, hydrogel monomer - Lubrizol, AMPS 2411
1-hydroxy cyclo hexyl phenyl ketone (99%); photoinitiator - Aldrich: 40561-2
Ebecryl 11 (PEG 400 diacrylate); crosslinker - UCB Chemicals
Potassium nitrite (>98%) -Fluka: 60417
L-Glutathione, reduced (>99%) - Sigma:G4251
Glucose - Fisher - analytical grade, code G050061
Glucose Oxidase - Biocatalysts - G638P (~70U /mg solid)
PVA (polyvinyl alcohol, Mᵣ = 124,000 to 186,000, 98-99% hydrolysed) - Aldrich: 36316-2

### Poly-AMPS hydrogel preparation

The components were mixed in the combinations and quantities indicated in Table 1, following the basic procedure set out below:
Stock solutions (as supplied by the manufacturer) of ammonium AMPS and/or sodium AMPS were dispensed into a 250 ml polypropylene, screw-top reaction jar as the basis of the pre-gel fluid. Glucose oxidase and the additive(s) (if required) were added to the mixture and allowed to dissolve completely. In a separate vessel the photoinitiator powder was dispersed in the liquid cross-linker and the mixture was warmed gently to dissolve the photoinitiator intro the cross-linker. This solution was then mixed into the pre-gel fluid. To cast the gels, the complete pre-gel fluid was poured into a flat bottomed tray, to a depth of 1 - 2mm. The gels were set by UV irradiation from a 1 kW lamp, at a vertical distance of 15 cm, for 25 seconds. The gels were allowed to cool before use.

**Table 1. Composition of hydrogels used in the study.**

| **Component** | | **Concentration of the stock solution (w/w)** | **Concentration in the final gel (w/w)** |
|---|---|---|---|
| | | | |
| ***Blank gel*** | | | |
| | Sodium AMPS | 50% aq | 30% |
| | Cross-linker | undiluted | 0.20% |
| | Photoinitiator | undiluted | 0.01 % |
| | Water | | to total weight |
| | | | |
| ***Enzyme (Glucose oxidase) gel*** | | | |
| | Sodium AMPS | 50% aq | 15% |
| | Ammonium AMPS | 50% aq | 15% |
| | Cross-linker | undiluted | 0.20% |
| | Photoinitiator | undiluted | 0.01 % |
| | Glucose oxidase | solid powder | 350 ug/g |
| | Water | | to total weight |
| | | | |
| ***Nitrite gel*** | | | |
| | Sodium AMPS | 50% aq | 30% |
| | Cross-linker | undiluted | 0.20% |
| | Photoinitiator | undiluted | 0.01 % |
| | Potassium nitrite | | 0.25% (= approx. 30 mM) |
| | Water | | to total weight |
| | | | |
| ***NitritelGlucose gel*** | | | |
| | | | |
| | | | |
| | Sodium AMPS | 50% aq | 30% |
| | Cross-linker | undiluted | 0.20% |
| | Photoinitiator | undiluted | 0.01% |
| | Glucose | solid powder | 5% |
| | Potassium nitrite | | 0.25% (= approx. 30 mM) |
| | Water | | to total weight |

### Preparation of the PVA layers

A stock solution of PVA (5% w/w) was prepared in water. Either potassium nitrite and/or L-glutathione were added to the PVA solution to achieve the required concentration (see Table 2). The solution was then dispensed into a Petri-dish (12 g over 60 cm²) and dried at 40°C overnight.

**Table 2. Composition of PVA layers used in the study. The composition is of the PVA-based mixtures before drying.**

| **Component** | | **Concentration of the aqueous stock solution (w/w)** | **Concentration in the final mixture (w/w)** |
|---|---|---|---|
| | | | |
| ***Glutathione layer*** | | | |
| | PVA | 5% aq | 4.5% |
| | L-Glutathione | 300 mM | 30 mM |
| | Water | | to total weight |
| | | | |
| ***Glutathione*/*Nitrite layer*** | | | |
| | PVA | 5% aq | 4% |
| | L-Glutathione | 300 mM | 30 mM |
| | Potassium nitrite | 300 mM | 30 mM |
| | Water | | to total weight |

### Measurement of S-nitrosoglutathione concentration

### Measurement of S-nitrosoglutathione concentration in aqueous solutions

The following reagents were prepared:
- Reagent 1:: Na-phosphate buffer (pH 7.4, 0.1 M)

- Reagent 2:: Griess reagent: 20 mg of N-(1-Naphthyl)ethylendiamine dihydrochloride (NADD) + 500 mg of sulphanilamide dissolved in 2 mL of DMSO. (N.B. This solution is light sensitive and should be kept in the dark as much as possible)
- Reagent 3:: Mercuric chloride (10 mM) in DMSO (13.58 mg of HgCl₂ in 5 mL of DMSO)

The six-step procedure set out below was then followed:
1. Dispense 1.5 mL of Reagent 1 into a plastic cuvette
2. Add 200 µL of the sample (i.e. sample in which GSNO concentration is to be determined)
3. Add 1.17 mL of DI water
4. Add 100 µL of Reagent 2
5. Add 30 µL of Reagent 3 and give the solution a good mix
6. Read absorbance of the resulting mixture at 496 nm in 10 min.

The concentration of GSNO can be calculated from the absorbance reading using the molar absorption coefficient for GSNO = 12,500 M¹cm⁻¹.

### Measurement of S-nitrosoglutathione concentration in hydrogels

The following reagents were prepared:
- Reagent 1:: Na-phosphate buffer (pH 7.4, 0.1 M)
- Reagent 2:: Griess reagent: 20 mg of N-(1-Naphthyl)ethylendiamine dihydrochloride (NADD) + 500 mg of sulphanilamide dissolved in 2 mL of DMSO. (N.B. This solution is light sensitive and should be kept in the dark as much as possible)
- Reagent 3:: Mercuric chloride (10 mM) in DMSO (13.58 mg of HgCl₂ in 5 mL of DMSO)

The five-step procedure set out below was then followed:
1. Dispense 25 mL of Reagent 1 and 825 µL of Reagent 2 into a 250 ml polypropylene pot.
2. Weigh precisely approximately 300 mg of the gel and immerse it in the reagent mix. Incubate while shaking mildly for 30 min.
3. Transfer 2.6 mL of the reagent mix from the polypropylene pot into a plastic cuvette
4. Add 25 µL of Reagent 3
5. Read absorbance of the resulting mixture at 496 nm in 10 min.

The concentration of GSNO in the reagent mix can be calculated from the absorbance reading using the molar absorption coefficient for GSNO = 12,500 M⁻¹ cm⁻¹ This can then be used to calculate the original concentration of GSNO in the gel.

### Example 1: Effect of pH on the rate of production/degradation of GSNO in aqueous system containing L-glutathione and potassium nitrite

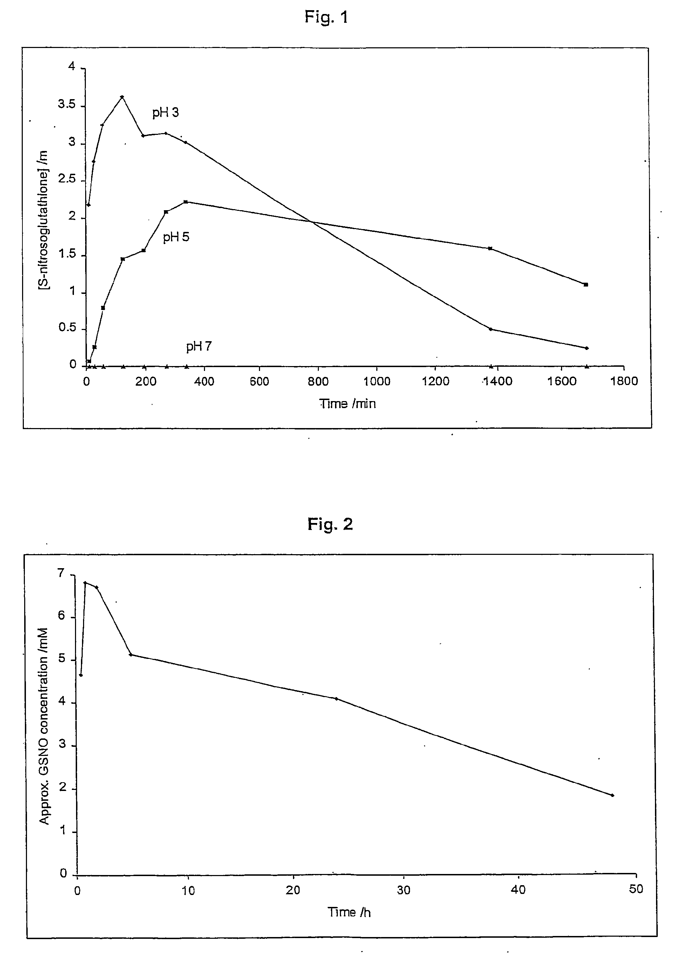

The rate of the GSNO production and subsequent decomposition in solutions containing potassium nitrite (5 mM) and L-glutathione (5 mM) was studied at pH 3 (citrate-phosphate buffer, 0.1 M), pH 5 (citrate-phosphate buffer, 0.1 M) and pH 7 (phosphate buffer, 0.1 M). The results are shown in Figure 1. No production of GSNO was observed at pH 7. The initial production of GSNO was slower at pH 5 compared with pH 3. The stability of GSNO produced appeared to be slightly higher at pH 5 compared with that at pH 3.

### Example 2: Generation of S-nitrosoglutathione in (unbuffered) activated hydrogel dressing

The concentration profile of GSNO in the activated dressing was measured in the absence of additional source of protons. The primary layer of the dressing consisted of poly-AMPS hydrogel containing potassium nitrite (30 mM). The secondary layer consisted of dried PVA (5%) containing L-glutathione (30 mM). There was no additional source of protons incorporated in the dressing. The layers were brought together to activate the dressing, and the results are shown in Figure 2. The concentration of GSNO generated in the dressing peaked after approximately 2 hours. Then there was a slow steady decline in the concentration of GSNO due to its slow decomposition. GSNO was still measurable in the dressing 48 hours after the activation.

### Example 3: Release of S-nitrosoglutathione from activated hydrogel dressing (with no additional source of protons incorporated) into a blank hydrogel

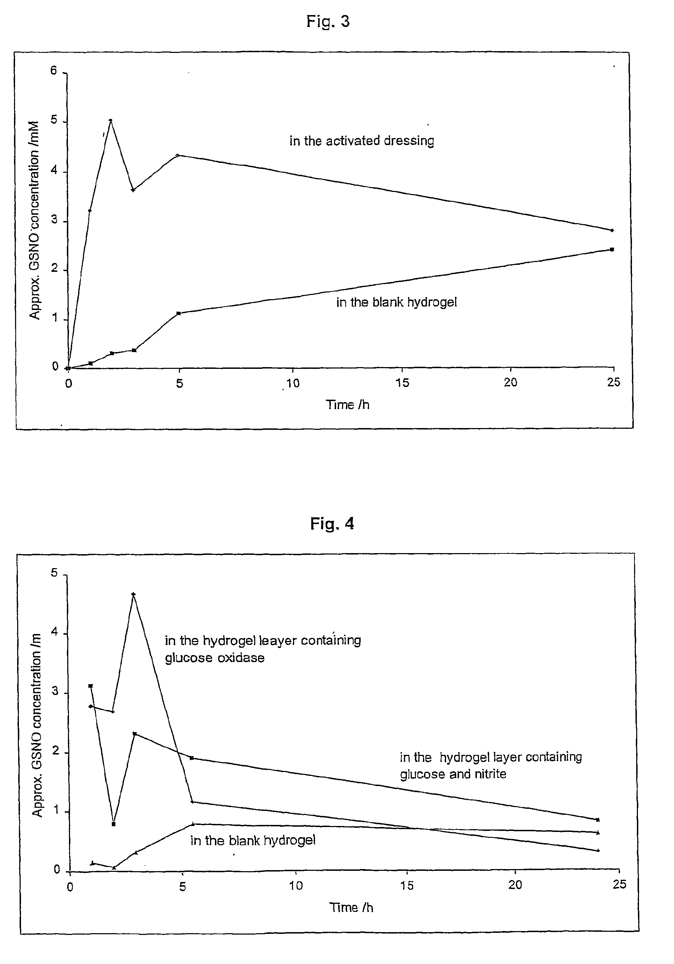

The primary layer of the dressing consisted of poly-AMPS hydrogel containing potassium nitrite (30 mM). The secondary layer consisted of dried PVA (5%) containing L-glutathione (30 mM). There was no additional source of protons incorporated in the dressing. The dressing was activated by bringing together the primary and the secondary layer. The activated dressing was placed onto a blank piece of hydrogel (30% poly-AMPS). The generation of GSNO inside of the dressing and its gradual release into the blank hydrogel was measured, and the results are shown in Figure 3.

The concentration of GSNO in the activated dressing peaked approximately 2 hours after the dressing was activated. Then there was a slow steady decline in the concentration of GSNO due to its slow decomposition. A slow gradual release of GSNO from the activated dressing into the blank hydrogel was demonstrated. The concentration of GSNO in the blank hydrogel almost equilibrated with that in the dressing in about 25 hours.

### Example 4: Release of S-nitrosoglutathione from activated hydrogel dressing (with glucose oxidase/glucose system as the additional source of protons incorporated) into a blank hydrogel

The dressing consisted of three layers: The primary layer consisted of a polymeric matrix of poly-AMPS hydrogel which contained potassium nitrite (30 mM) and glucose (5% W/W). The secondary layer consisted of dried PVA (5%) containing L-glutatbione (30 mM). The tertiary layer consisted of a polymeric matrix of poly-AMPS hydrogel which contained glucose oxidase (0.035% w/w). The dressing was activated by bringing all three layers together in the arrangement where the secondary layer is sandwiched between the primary and the tertiary layer. The activated dressing was placed onto a blank piece of hydrogel (30% poly-AMPS). The generation of GSNO inside of the dressing and its gradual release into the blank hydrogel was measured, and the results are shown in Figure 4.

The concentration profile of GSNO in the dressing and in the blank hydrogel was very similar to that observed in the absence of additional source of protons (see Example 3). The concentration of GSNO in the activated dressing peaked approximately 2 hours after the dressing was activated. Then there was a slow steady decline in the concentration of GSNO due to its slow decomposition. A slow gradual release of GSNO from the activated dressing into the blank hydrogel was demonstrated.

### Example 5: Release of S-nitrosoglutathione from dried PVA layer containing pre-generated S-nitrosoglutathione

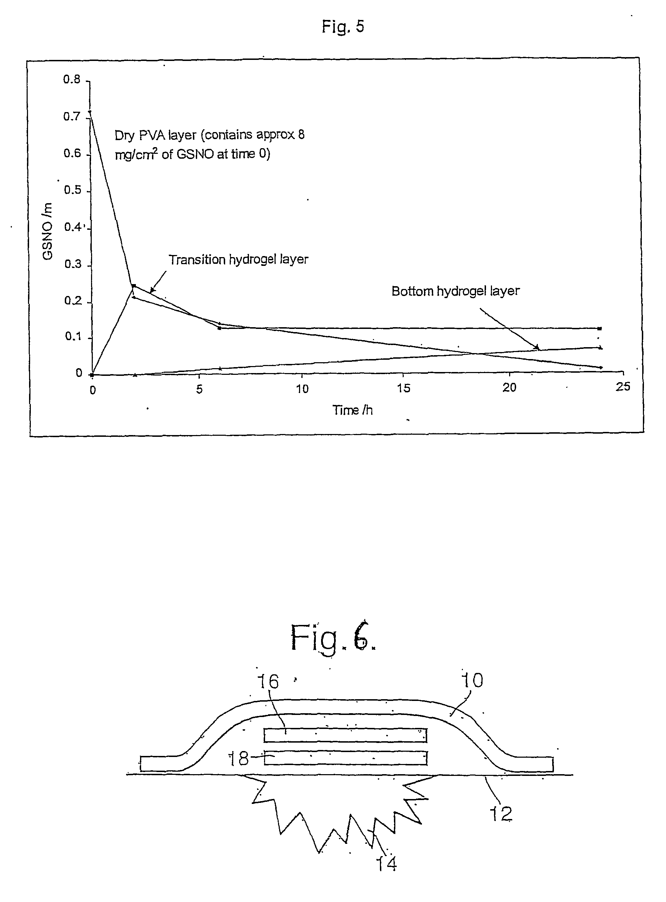

A dry PVA layer containing S-nitrosoglutathione was prepared by mixing 9 mL of PVA solution (5% w/w) with 1 mL of L-Glutathione (300 mM) and 1 mL of potassium nitrite (300 mM). The mixture was dried on a Petri dish (60 cm²) at 40 °C for 5 h. This resulted in a dry film containing approximately 8 mg of S-nitrosoglutathione per cm². The release of S-nitrosoglutahione from this film was demonstrated by placing 1 cm² of the film onto two layers of blank poly-AMPS (30% w/w) hydrogel and measuring GSNO in all three layers at given time-points (2 h, 6 h and 24 h after applying the film). Whilst the first hydrogel layer (i.e. the one in contact with GSNO-containing film) served as the transition layer the second hydrogel (beneath the transition layer) was used to mimic the surrounding environment such as wound bed. The rate of release of GSNO is shown in Figure 5.

GSNO was released rapidly from the GSNO-containing film into the transition layer where the GSNO concentration peaked at approximately 2 h. GSNO was detectable in the bottom hydrogel several hours later and its concentration kept increasing gradually. The total amount of GSNO detectable in the entire system was gradually declining due to slow decomposition of GSNO.

Figure 6 illustrates schematically a skin dressing in accordance with the invention.

The illustrated dressing is of layered construction and comprises an optional outer layer or covering 10 in the form of a plaster suitable for adhering to the skin 12 of a subject, so as to cover a wound 14. Covering 10 encloses a first component or primary layer 18 and a second component or secondary layer 16.

The first component 18 comprises a layer of poly-AMPS hydrogel incorporating potassium nitrite, in the form of a nitrite gel as specified in Table 1 above. The second component 16 comprises a layer of dried PVA incorporating L-glutathione, in the form of a glutathione layer as specified in Table 2 above.

The dressing is initially supplied as a multi-part system, with the individual components separately packaged in respective sealed, sterile packages. When required for use, the dressing components are removed from the packages and applied to a wound in appropriate manner and order to produce the final dressing as shown. When the first and second components are brought together, this activates the gel.

On activation, nitrite starts diffusing from the primary layer into the secondary layer and the thiol diffuses in the opposite direction. Mixing of the nitrite with the thiol results in generation of S-nitrosoglutathione (GSNO) Once produced, the GSNO is released from the dressing into the surrounding environment where it decomposes to produce nitric oxide.

### Example 6: a delivery system for the generation of nitric oxide

GSNO is not suitable for long term storage because it decomposes readily to release NO. Using a dual dressing component storage and dispensing configuration allows separate storage of reactants which, when dispensed, are mixed to initiate the reaction to generate S-nitrosoglutathione.

The first component was prepared as follows: the base carrier used to add viscosity and spreadability was a hydrogel based material, commercially named Plexajel ASC (United Guardian Inc.). Into this, phosphate buffered saline was diluted 1/20 from a 10x concentrated stock (Sigma, D1408). Potassium nitrite (Fluka, 60417) was added to give a final concentration of 60mM and allowed to dissolve.

The second component was prepared as follows: L-glutathione (reduced form Sigma, G4251) was suspended in propylene glycol (Fluka 82281) to give a final concentration equivalent to 60mM.

Both components were stored separately. A dual dispenser was obtained from Versdial Inc, which allows variable control of the dispensing volumes from the two chambers. Samples came into contact with each other only when they were dispensed from their respective isolate chambers.

To demonstrate the effect of NO generated from the two separately stored reagents, blood flow to the surface of the forearm skin was investigated using laser Doppler. A Moor Instruments DRT4 tissue blood-flow and temperature monitor with associated skin probes was used to measure the skin laser Doppler flux. Two probes were attached to the skin, positioned to avoid major veins and arteries, at approximately 5-10cm apart. The instrument was run for 1min to ensure a flat response. One of the chambers was filled with nitrite and the other with L-glutathione. As a control, a second set of chambers were filled with water. The nitrite and L-glutathione were mixed just prior to use, in equal quantities. The mix was stirred to ensure the L-glutathione particles within the propylene glycol were dissolved in the aqueous nitrite hydrogel. The skin laser Doppler flux was then measured until a plateau was observed, indicating the maximum vaso-dilation effect had been reached.

Table 1 demonstrates the vaso-dilation effect on the skin; when using the 2 component system. The LDF value for the mixture of L-glutathione and nitrite begins to increase after 2 minutes (post sample application) indicating the speed of NO production and dermal transmission. The water control remains flat thus indicating the increase in blood flow to be due to the nitrite/GSH reaction. The maximal response of approximately 150 LDF units is regarded as being a strong response.

**Table 1**

| Time (mins) | Layer Doppler Flux | |
|---|---|---|
| | Test | control |
| 0 | 29.3 | 45.6 |
| 1 | 13.7 | 24.3 |
| 2 | 30.3 | 31.2 |
| 3 | 34.6 | 35.0 |
| 4 | 52.0 | 33.3 |
| 5 | 85.2 | 42.7 |
| 6 | 108.3 | 34.5 |
| 7 | 161.4 | 35.0 |
| 8 | 151.6 | 30.3 |
| 9 | 165.3 | 33.7 |

## Claims

1. A skin dressing adapted, on activation, to release one or more S-nitrosothiols, wherein the one or more S-nitrosothiols are generated by reacting together reagents, comprising a nitrite and a thiol, in the dressing.

2. A dressing according to claim 1, wherein the dressing comprises one or more dressing components.

3. A dressing according to claim 2, wherein the or each dressing component is in the form of a layer.

4. A dressing according to claim 2 or 3, wherein the or each dressing component comprises a carrier or support.

5. A dressing according to claim 4, wherein the carrier or support comprises a polymeric matrix.

6. A dressing according to claim 5, wherein the polymeric matrix comprises a hydrated hydrogel.

7. A dressing according to claim 6, wherein the hydrated hydrogel comprises poly 2-acrylamido-2-methylpropane sulphonic acid (poly-AMPS) and/or salts thereof.

8. A dressing according to claim 5, wherein the polymeric matrix comprises a dried polymer.

9. A dressing according to claim 8, wherein the polymer comprises polyvinyl alcohol.

10. A dressing according to any one of the preceding claims, wherein the nitrite comprises potassium nitrite.

11. A dressing according to any one of the preceding claims, wherein the thiol comprises glutathione, preferably L-glutathione.

12. A dressing according to any one of the preceding claims, wherein the reagents react together in the dressing on activation to generate and release the one or more S-nitrosothiols.

13. A dressing according to claim2, wherein the dressing comprises a first dressing component comprising a nitrite and a second dressing component comprising the thiol.

14. A dressing according to claim 13, wherein the first dressing component comprises a hydrated hydrogel and the second dressing component comprises a dried polymeric matrix.

15. A dressing according to claim 14, wherein hydrated hydrogel comprises poly-AMPS and/or salts thereof, and the dried polymeric matrix comprises dried polyvinyl alcohol.

16. A dressing according to any one of claims 12 to 15, wherein the dressing includes and/or generates on activation a source of protons.

17. A dressing according to claim 16, including an acid or acidic buffer in one or more dressing components.

18. A dressing according to claim 16 or 17, including an oxidase enzyme and substrate therefor.

19. A dressing according to claim 18, wherein the enzyme is glucose oxidase and the substrate is glucose.

20. A dressing according to claim 18 or 19 when dependent on claim 13, 14, 15, wherein the first dressing component includes the substrate.

21. A dressing according to claim 20, wherein the second dressing component includes the enzyme.

22. A dressing according to claim 20, wherein the enzyme is included in a third dressing component.

23. A dressing according to any one of claims 1 to 11, wherein the reagents react together in the dressing to generate the one or more S-nitrosothiols before activation to release the one or more S-nitrosothiols.

24. A dressing according to claim 23, including S-nitrosothiol in dry, inactive condition.

25. A dressing according to claim 24, wherein the S-nitrosothiol is present in a dressing component comprising a dried polymeric matrix.

26. A dressing according to claim 25, wherein the dressing component comprises dried polyvinyl alcohol.

27. A dressing according to any one of the preceding claims, wherein the S-nitrosothiol comprises S-nitrosoglutathione.

28. A dressing according to any one of the preceding claims, comprising two components which are amorphous.

29. A dressing according to any one of the preceding claims, including or for use with a skin contact layer comprising a hydrated hydrogel of poly-AMPS and/or salts thereof.

30. A dressing according to any one of the preceding claims, wherein the or each dressing component is stored in a respective package prior to use.

31. A method of generating nitric oxide for cosmetic purposes on or in the vicinity of a body surface, the method comprising reacting a nitrite and a thiol in a dressing to generate one or more S-nitrosothiols which decompose spontaneously to deliver the nitric oxide.

## Patentansprüche

1. Hautverband, dafür ausgelegt, bei Aktivierung ein oder mehrere S-Nitrosothiole freizusetzen, wobei das eine oder die mehreren S-Nitrosothiole durch miteinander Umsetzen von Reagenzien, die ein Nitrit und ein Thiol umfassen, in dem Verband erzeugt werden.

2. Verband gemäß Anspruch 1, wobei der Verband eine oder mehrere Verbandkomponenten umfasst.

3. Verband gemäß Anspruch 2, wobei die oder jede Verbandkomponente in der Form einer Schicht vorliegt.

4. Verband gemäß Anspruch 2 oder 3, wobei die oder jede Verbandkomponente einen Träger oder eine Stütze umfasst.

5. Verband gemäß Anspruch 4, wobei der Träger oder die Stütze eine polymere Matrix umfasst.

6. Verband gemäß Anspruch 5, wobei die polymere Matrix ein hydratisiertes Hydrogel umfasst.

7. Verband gemäß Anspruch 6, wobei das hydratisierte Hydrogel Poly-2-acrylamido-2-methylpropansulfonsäure (poly-AMPS) und/oder Salze davon umfasst.

8. Verband gemäß Anspruch 5, wobei die polymere Matrix ein getrocknetes Polymer umfasst.

9. Verband gemäß Anspruch 8, wobei das Polymer Polyvinylalkohol umfasst.

10. Verband gemäß einem der vorstehenden Ansprüche, wobei das Nitrit Kaliumnitrit umfasst.

11. Verband gemäß einem der vorstehenden Ansprüche, wobei das Thiol Glutathion, vorzugsweise L-Glutathion, umfasst.

12. Verband gemäß einem der vorstehenden Ansprüche, wobei sich die Reagenzien bei Aktivierung in dem Verband miteinander umsetzen, um das eine oder die mehreren S-Nitrosothiole zu erzeugen und freizusetzen.

13. Verband gemäß Anspruch 12, wobei der Verband eine erste Verbandkomponente, umfassend ein Nitrit, und eine zweite Verbandkomponente, umfassend das Thiol, umfasst.

14. Verband gemäß Anspruch 13, wobei die erste Verbandkomponente ein hydratisiertes Hydrogel umfasst und die zweite Verbandkomponente eine getrocknete polymere Matrix umfasst.

15. Verband gemäß Anspruch 14, wobei das hydratisierte Hydrogel poly-AMPS und/oder Salze davon umfasst, und die getrocknete polymere Matrix getrockneten Polyvinylalkohol umfasst.

16. Verband gemäß einem der Ansprüche 12 bis 15, wobei der Verband eine Protonenquelle umfasst und/oder bei Aktivierung erzeugt.

17. Verband gemäß Anspruch 16, umfassend eine Säure oder einen sauren Puffer in einer oder mehreren Verbandkomponenten.

18. Verband gemäß Anspruch 16 oder 17, umfassend ein Oxidaseenzym und Substrat dafür.

19. Verband gemäß Anspruch 18, wobei das Enzym Glucoseoxidase ist und das Substrat Glucose ist.

20. Verband gemäß Anspruch 18 oder 19, wenn abhängig von Anspruch 13, 14, 15, wobei die erste Verbandkomponente das Substrat umfasst.

21. Verband gemäß Anspruch 20, wobei die zweite Verbandkomponente das Enzym umfasst.

22. Verband gemäß Anspruch 20, wobei das Enzym in einer dritten Verbandkomponente enthalten ist.

23. Verband gemäß einem der Ansprüche 1 bis 11, wobei sich die Reagenzien in dem Verband miteinander umsetzen, um die eine oder mehreren S-Nitrosothiole vor der Aktivierung zum Freisetzen der einen oder mehreren S-Nitrosothiole zu erzeugen.

24. Verband gemäß Anspruch 23, umfassend S-Nitrosothiol in trockenem, inaktiven Zustand.

25. Verband gemäß Anspruch 24, wobei das S-Nitrosothiol in einer Verbandkomponente vorliegt, die eine getrocknete polymere Matrix umfasst.

26. Verband gemäß Anspruch 25, wobei die Verbandkomponente getrockneten Polyvinylalkohol umfasst.

27. Verband gemäß einem der vorstehenden Ansprüche, wobei das S-Nitrosothiol S-Nitrosoglutathion umfasst.

28. Verband gemäß einem der vorstehenden Ansprüche, umfassend zwei Komponenten, die amorph sind.

29. Verband gemäß einem der vorstehenden Ansprüche, umfassend eine oder zur Verwendung mit einer Hautkontaktschicht, die ein hydratisiertes Hydrogel aus poly-AMPS und/oder Salzen davon umfasst.

30. Verband gemäß einem der vorstehenden Ansprüche, wobei die oder alle Verbandkomponenten vor der Verwendung in einer entsprechenden Verpackung gelagert sind.

31. Verfahren zum Erzeugen von Stickoxid für kosmetische Zwecke auf oder in der Nähe einer Körperoberfläche, wobei das Verfahren das Umsetzen eines Nitrits und eines Thiols in einem Verband zum Erzeugen von einem oder mehreren S-Nitrosothiolen, die sich spontan zur Abgabe des Stickoxids zersetzen, umfasst.

## Revendications

1. Pansement cutané adapté, sur l'activation, à libérer un ou plusieurs S-nitrosothiols, **caractérisé en ce que** le ou lesdits un ou plusieurs S-nitrosothiols sont générés en faisant réagir ensemble des réactifs, comprenant un nitrite et un thiol, dans le pansement.

2. Pansement selon la revendication 1, **caractérisé en ce que** le pansement comprend un ou plusieurs composants de pansement.

3. Pansement selon la revendication 2, **caractérisé en ce que** ledit ou chaque composant du pansement est sous forme d'une couche.

4. Pansement selon la revendication 2 ou 3, **caractérisé en ce que** ledit ou chaque composant du pansement comprend un véhicule ou un support.

5. Pansement selon la revendication 4, **caractérisé en ce que** le véhicule ou le support comprend une matrice polymère.

6. Pansement selon la revendication 5, **caractérisé en ce que** la matrice polymère comprend un hydrogel hydraté.

7. Pansement selon la revendication 6, **caractérisé en ce que** l'hydrogel hydraté comprend l'acide poly-2-acrylamido-2-méthylpropanesulfonique (poly-AMPS) et/ou des sels de celui-ci.

8. Pansement selon la revendication 5, **caractérisé en ce que** la matrice polymère comprend un polymère séché.

9. Pansement selon la revendication 8, **caractérisé en ce que** le polymère comprend de l'alcool polyvinylique.

10. Pansement selon l'une quelconque des revendications précédentes, **caractérisé en ce que** le nitrite comprend le nitrite de potassium.

11. Pansement selon l'une quelconque des revendications précédentes, **caractérisé en ce que** le thiol comprend le glutathion, de préférence le L-glutathion.

12. Pansement selon l'une quelconque des revendications précédentes, **caractérisé en ce que** les réactifs réagissent ensemble dans le pansement sur activation pour générer et libérer le ou lesdits un ou plusieurs S-nitrosothiols.

13. Pansement selon la revendication 12, **caractérisé en ce que** le pansement comprend un premier composant du pansement comprenant un nitrite et un deuxième composant du pansement comprenant le thiol.

14. Pansement selon la revendication 13, **caractérisé en ce que** le premier composant du pansement comprend un hydrogel hydraté et le deuxième composant du pansement comprend une matrice polymère séchée.

15. Pansement selon la revendication 14, **caractérisé en ce que** l'hydrogel hydraté comprend le poly-AMPS et/ou des sels de celui-ci, et la matrice polymère séchée comprend de l'alcool polyvinylique séché.

16. Pansement selon l'une quelconque des revendications 12 à 15, **caractérisé en ce que** le pansement inclut et/ou génère sur activation une source de protons.

17. Pansement selon la revendication 16, comportant un acide ou un tampon acide dans un ou plusieurs composants du pansement.

18. Pansement selon la revendication 16 ou 17, comportant une enzyme oxydase et un substrat de celle-ci.

19. Pansement selon la revendication 18, **caractérisé en ce que** l'enzyme est le glucose oxydase et le substrat est le glucose.

20. Pansement selon la revendication 18 ou 19, lorsqu'elles dépendent de la revendication 13, 14 ou 15, **caractérisé en ce que** le premier composant du pansement inclut le substrat.

21. Pansement selon la revendication 20, **caractérisé en ce que** le deuxième composant du pansement inclut l'enzyme.

22. Pansement selon la revendication 20, **caractérisé en ce que** l'enzyme est incluse dans un troisième composant du pansement.

23. Pansement selon l'une quelconque des revendications 1 à 11, **caractérisé en ce que** les réactifs réagissent ensemble dans le pansement pour générer le ou lesdits un ou plusieurs S-nitrosothiols avant l'activation pour libérer un ou plusieurs S-nitrosothiols.

24. Pansement selon la revendication 23, comportant du S-nitrosothiol à l'état sec inactif.

25. Pansement selon la revendication 24, **caractérisé en ce que** le S-nitrosothiol est présent dans un composant du pansement comprenant une matrice polymère séchée.

26. Pansement selon la revendication 25, **caractérisé en ce que** le composant du pansement comprend de l'alcool polyvinylique séché.

27. Pansement selon l'une quelconque des revendications précédentes, **caractérisé en ce que** le S-nitrosothiol comprend du S-nitrosoglutathion.

28. Pansement selon l'une quelconque des revendications précédentes, comprenant deux composants qui sont amorphes.

29. Pansement selon l'une quelconque des revendications précédentes, comportant ou destiné à être utilisé avec une couche de contact de la peau comprenant un hydrogel hydraté de poly-AMPS et/ou des sels de celui-ci.

30. Pansement selon l'une quelconque des revendications précédentes, **caractérisé en ce que** ledit ou chaque composant du pansement est stocké dans un emballage respectif avant l'utilisation.

31. Méthode de génération d'oxyde nitrique à des fins cosmétiques sur ou au voisinage d'une surface du corps, la méthode comprenant la réaction d'un nitrite et d'un thiol dans un pansement pour générer un ou plusieurs S-nitrosothiols qui se décomposent spontanément pour délivrer l'oxyde nitrique.
